Europäisches Patentamt

European Patent Office

Office européen des brevets

(19)

(11) Publication number: **0 159 054**
**B1**

(12) **EUROPEAN PATENT SPECIFICATION**

(45) Date of publication of patent specification: **25.01.89**

(51) Int. Cl.⁴: **C 12 P 5/02**

(21) Application number: **85200332.6**

(22) Date of filing: **07.03.85**

(54) **A process for producing methane from solid vegetable material.**

(30) Priority: **09.03.84 NL 8400764**

(43) Date of publication of application:
**23.10.85 Bulletin 85/43**

(45) Publication of the grant of the patent:
**25.01.89 Bulletin 89/04**

(84) Designated Contracting States:
**AT BE CH DE FR GB IT LI LU NL SE**

(56) References cited:
**US-A-3 711 392**
**US-A-4 127 447**
**US-A-4 318 993**

(73) Proprietor: **Stichting Katholieke Universiteit**
**Toernooiveld 1**
**NL-6525 ED Nijmegen (NL)**

(72) Inventor: **Vogels, Godefridus Dionisius**
**Schepenenstraat 2**
**Nijmegen (NL)**

(74) Representative: **Urbanus, Henricus Maria, Ir.**
**et al**
**c/o Vereenigde Octrooibureaux Nieuwe**
**Parklaan 107**
**NL-2587 BP 's-Gravenhage (NL)**

## Description

This invention relates to a process for producing methane from solid vegetable material.

Unlike the anaerobic purification of dissolved organic waste, which generally proceeds very well, there is not as yet an effective system for the conversion of solid vegetable waste material into biogas. Existing solids fermenters generally operate rather inefficiently. To give an example: In manure fermenters, degradation on COC basis (Chemical Oxygen Consumption) is approximately 60% with a residence time of the manure of 10 to 20 days and a load of 1—5 kg COC/$m^3$. day. Methane production is then approximately 2 $m^3/m^3$. day. This means that the conversion of a given waste stream requires relatively large fermenters (low loads and long residence times).

In the rumen of ruminants (cow, sheep, etc.), solid vegetable material (grass) is very efficiently converted into methane and volatile fatty acids. In this process, a unique system of micro-organisms is involved (ruminal ciliates and bacteria). The residence time of grass in the rumen is approximately 30 hours with a load (converted) of about 40 kg COC/$m^3$. day. Degradation is then approximately 70%. A similar system is found in the cecum of herbivores.

The anaerobic degradation of organic material takes place in four phases.

The polymers formed during photosynthesis are converted into monomers, such as glucose (phase I), from which a large number of fermentation products are formed (phase II). These fermentation products are converted by acetate and/or hydrogen forming micro-organisms into acetic acid, hydrogen and $CO_2$ (phase III), which substances are, in turn, the specific nutrients for methane forming bacteria (phase IV). This process takes place, for example, in the rumen of ruminants. A not inconsiderable portion of the product of photosynthesis (estimated proportion 5 to 10%) is converted in this manner; the methane thus formed is converted by methane oxidizing bacteria or by reactions in the ozone layer of the atmosphere into $CO_2$ and thus again becomes available for photosynthesis.

The anaerobic degradation process of organic material can also take place without the participation of methane forming bacteria. The micro-organisms of phase III then produce acetic acid and/or hydrogen. In the absence of methane bacteria, however, the medium will acidify, and hydrogen pressure will rise. Hydrogen is an inhibitory product of anaerobic metabolism: the enzyme involved in the hydrogen formation, hydrogenase, often operates in a reversible manner, whereby energy supplying oxidations are frustrated when hydrogen accumulates.

In the presence of methane bacteria, metabolic control of the entire ecosystem takes place via three mechanisms:

1. a proton control, in which acid products (acetic acid and other acids) are converted into the inert methane and the weakly acidic $CO_2$;

2. an electron control, whereby the methane bacteria make for the formation and further conversion of hydrogen (from $H^+$), with the result that the organisms of phase III and IV can win energy for their growth and also that the organic material is fully converted into inert materials under anaerobic conditions;

3. a nutrient control, which relates not only to the throughflow of degradational intermediates, but also to the supply of growth factors (e.g. $B_{12}$ compounds) to the other organisms, including the animal host, if any.

The presence of methane bacteria prevents the accumulation of acids and the inhibitory hydrogen, and results in the formation of an inert product that is easily separated, namely, the methane.

Existing ecosystems can be classed in two groups, viz:

a. all freshwater systems, including installations for biogas production and anaerobic purification, and

b. salt-water and gastrointestinal systems.

In the ecosystems of group a (hereinafter referred to as the *acetate systems*), the methane is formed as to about 70% from acetate, whereas in the systems of group b (hereinafter referred to as the *hydrogen systems*), the methane is formed virtually completely from hydrogen and $CO_2$. The salt-water systems, in which sulphate-reducing bacteria play an important role, will be left out of account herein. Among the gastrointestinal systems, the rumens of ruminants are most prominent with an annual production of 150—300×$10^9$ $m^3$ methane.

The acetate systems are further characterized by relatively slow rates of throughflow of the system ("vat cycles" of one week to several months), whereas the hydrogen systems are relatively fast ("vat cycles" of one or several days). Possibly this is due to the fact that the methane fermentation is the rate-limiting step and that methane bacteria are capable of extracting considerably more energy from hydrogen+$CO_2$ (change in free energy −131 KJ/mole methane) than from acetic acid (change in free energy −31 KJ/mole methane).

From these data it could be concluded that, in nature, often a slowly proceeding anaerobic acetate system will establish itself in the decomposition of organic material (comparable to the stable state), but that in addition a fast process may establish itself in countless hydrogen systems with a relatively large contribution (20% of the total) to the annual production on earth (comparable to the metastable state). The use of the hydrogen system may therefore have great advantages, if the conditions for the stabilization of the system are known.

We will now discuss a number of features of the ruminal system of ruminants, especially with a view to facts which are of importance for an understanding of the occurrence of an evidently rather stable hydrogen system with a relatively high rate of throughflow.

The rumen of ruminants always contains large numbers (100,000 cells per ml) of ciliates (about 11 species belonging to 9 genera). The contribution of the ciliates to the contents of the rumen can be derived, for one thing, from the fact that often more than 40% (sometimes even 80%) of the nitrogen present in the ruman is contained in the ciliates. If only on the ground of the large numbers of ciliates and their considerable contribution to the population of micro-organisms, the ciliates may be expected to play an important role in the conversion of organic material.

The ciliate system is readily established (is readily taken over by the calf from the cow) and its general occurrence indicates an ecological stability evidently with various advantages for the system and the host over and above an acetate system.

The role of the ciliates can be deduced from a number of observations of phenomena in the rumen. It has been observed that, on the interfaces, the plant material is very rapidly occupied by ciliates, which take the fibrous cells in their buccal cavity. Around the engulfed piece of plant material, a number of largely unknown processes takes place within the ciliate. Probably the ciliate, or bacteria living (endosymbiotically) within the ciliate, produce the enzymes which digest the plant material, whereby the end products of phase II are formed. These fermentation products are converted by the microbodies present in the ciliate into hydrogen and $CO_2$.

The presence of hydrogen slows down the overall degradation process. Attached to the cell surface on the outside of the ciliate, however, are methane bacteria (specifically *Methanobrevibacter ruminantium*), which convert hydrogen and $CO_2$ into the inert methane. Methane bacteria contain several unique and highly fluorescent co-enzymes: $F_{420}$, a deazaflavine involved in the hydrogen and electron transfer, and the methanopterines, which are involved in $C_1$ metabolism. The presence of these substances forms the basis of the epifluorscence technique developed by Doddema and Vogels for the identification of methane bacteria in mixed populations (e.g. the rumen). We were able to observe the above-described association in the rumen of cows, immediately after slaughter, in the rumen of a fistulated sheep, and in laboraty cultures of ruminal ciliates; in the monocultures of ciliates, the association can result in a density of about 10,000 methane bacteria being attached per ciliate cell.

The attachment (symbiosis) has at least a twofold effect:

a. an effective hydrogen transfer, owing to which the ciliate is minimally inhibited by hydrogen (the hydrogen pressure in the rumen is $10^{-6}$ atm) and the methane bacteria find the food at the source;

b. via the complex (solid vegetable material—ciliates—attached methane bacteria) the methane bacteria benefit from a longer residence time in the rumen (necessary in connection with the long division time of the methane bacteria), and the ciliates benefit from a large number of methane bacteria present in their immediate vicinity.

The whole system forms a continuous culture with a throughflow time of about 1 day, buffered by bicarbonate from the saliva and the $CO_2$ formed during the decomposition, and is kept at a constant temperature by the ruminant. In spite of the heavy load on the system, the microorganisms are enabled to do the most difficult par of the job, namely, rendering the organic material accessible and degrading the polymers, within a short period of time. In the overall process, the ciliates/methane bacteria system plays an important role. The ruminant benefits indirectly: via the methane formed, 10% of the calorific value of the food is wasted, but the profit resides in the presence of an effective ecosystem which continuously provides the animal with the products of the decomposition of food that is difficult to digest by other animals.

It is an object of the present invention to provide a process with which solid vegetable material, in particular solid vegetable waste materials, for example industrial waste (paper mills, potato-flour mills, sugar factories, etc.), agrarian or domestic refuse, or also higher or lower plants (e.g. algae) specially cultured for the provision of energy, can be decomposed to form methane (biogas) in an efficient manner.

The process according to the invention is characterized by contacting the solid vegetable material in a reactor with a liquid system comprising at least one ciliate species and at least one species of methane bacterium, and removing a methane and $CO_2$ containing gas from the reactor.

A preferred embodiment is characterized by using at least one of the following ciliate species:

1. *Eudiplodinium maggii*
2. *Diplodinium dentatum*
   syn. *Diplodinium denticulatum f. anacanthum*
   syn. *D. denticulatum f. monacanthum*
3. *Epidinium ecaudatum*
   syn. *Epindium ecaudatum f. hamatum*
4. *Entodinium simplex*
5. *Entodinium longinudeatum*
6. *Entodinium caudatum*
7. *Dasytricha ruminantium*
8. *Isotricha prostoma*

It is further preferred to use at least the methane bacterium *Methanobrevibacter ruminantium*.

A preferred embodiment that is highly suitable in practice is characterized by using as the liquid system fluid (inoculating material) from the rumen of ruminants or the cecum of herbivores.

For optimum operation, it is preferred that the pH be maintained at a value of 5.5—7 and that the temperature be maintained at a value of 35—45°C, preferably at about 40°C.

In order for the system to function properly, preferably liquid is removed from the reactor continuously or discontinuously, and replaced by liquid which does not contain carboxylic acids or a lower proportion thereof.

In practice this means that at least 2 reactor volumes of liquid are removed and replaced per day.

In the most preferred embodiment, the system consists of two spatially separate phases, and is characterized in that the liquid removed from the reactor is subjected in a separate reactor to a treatment in which the carboxylic acids present in the liquid are decomposed to form methane and $CO_2$, which are removed from said second reactor, and the liquid thus treated is recycled to the first reactor or discharged.

In a further preferred embodiment, any remaining solid residue, which is rich in components that cannot be decomposed, or with great difficulty only, is removed from the first reactor after a residence time of the solid material of 1—3 days, and either dumped, or burnt, or used to recover valuable materials or as a fertilizer.

It is noted that it is known from US patent 3,711,392 to subject biodegradable waste materials, such as cellulose-containing materials, to the action of various microorganisms. The process comprises the use of known microbial processes for the conversion of biodegradable waste materials into various products which can serve as human or animal food. About the conversion, no quantitative data are given. The specification does state that a gaseous product comprising methane, hydrogen, carbon dioxide and ethanol is formed. Methane, however, is explicitly characterized as an undesirable byproduct, and the formation of methane is therefore preferably prevented by adding a small amount of carbon tetrachloride. Furthermore, various species of micro-organisms are mentioned which are suitable for use in this prior process. These do not, at any rate, include methane bacteria. The specification does mention protozoa, such as *Entoninium*, *Dasytricha* and *Diplodinium* rumen protozoa, but exclusively in connection with their capacity of rendering pathogenic bacteria harmless.

The process of the present invention is directed not to the production of food, but to the production of biogas by complete decomposition of vegetable material by means of a combination of organisms, among which ciliates and methane bacteria are essential. The ciliates are present in large numbers of e.g. 40 to as much as 80% of all nitrogen and, virtually proportionate thereto, of the total biomass, and lyse the vegetable material to form hydrogen, which is used for the methane production. The process of the invention uses the hydrogen system, whereas the process of the above US patent uses the relatively slow acetate system. Owing to the combination with methane bacteria, a virtually complete conversion of the vegetable material into methane and carbon dioxide is realized. Both liquid throughflow and the efficiency on the basis of solids (solids loading rate and residence time) are considerably higher than is the case in the process disclosed in US patent 3,711,392, while prefermentation is unnecessary. In addition, in one of the preferred embodiments of the present invention, byproducts are also converted into methane in a separate reactor, whereby the fluid thus treated is rendered suitable for being recycled to the first reactor. The present process does not include electrodialysis.

US patent specification 4,318,993 discloses a process for producing biogas from biodegradable materials, in which organic waste, after prehydrolysis and neutralization, is first decomposed in an acidification phase, and subsequently converted in a separate methane phase into biogas. No ciliates are used. In the process of the present invention, on the other hand, hydrolysis, acidification and the formation of hydrogen and methane take place simultaneously in the first reactor, which contains at least ciliates and methane bacteria. Remaining acids can then, if desired, be decomposed in a second reactor, which contains a known per se methane phase, to form an additional amount of methane gas. Accordingly, this process according to the invention does not include the prehydrolysis and neutralization described in the US patent. Some general advantages of methane formation over and above other systems in which the recovery of energy from organic material is a major object, are the following.

Methane fermentation produces a readily separated gas: the process does not require a major amount of energy in winning the energy-rich product, as is the case, for example, with alcohol production for energy purposes. The disadvantage of processes such as combustion and pyrolysis of the organic material is that its moisture content reduces effectiveness. Methane fermentation is accompanied by relatively little sludge growth; sludge produced is well-rotted and can often be directly used on the land as a fertilizer. In addition, the system permits heavy loads, which makes for a reduction in the cost of investment of the plant.

These and other advantages of the methane fermentation used also apply to the present system of ciliates and methane bacteria. This system further gives a good methane fermentation with typically vegetable and undissolved material, and permits relatively short throughflow times with relatively heavy loads.

These advantages are attributable to a splendid spatial organization in the ciliate: the systems involved in the digestion of the plant material and in the further degradation are concentrated within and on the cell, and the ciliate attends to tracing and supplying the food. Since a hydrogen system can establish itself for the methane formation, the methane bacteria get a larger share in the energy to be distributed, which promotes their growth (often the limiting step in the entire system).

The invention accordingly provides an applied system for the conversion of vegetable material into methane, which can be used as an important source of energy. The methane formed, which is in the gaseous form and readily separable, contains virtually all of the energy stored in the vegetable material during photosynthesis from the sun light. The digestion of the vegetable material (the plant cell) has hitherto been a barrier that was difficult to take in the conversion of plant material into methane, and often could only be accomplished by subjecting the vegetable material to chemical pretreatments.

The vegetable material may come from industries using vegetable or microbial raw materials and producing insoluble waste (e.g., papermills, various industries in the food and pharmaceutical sectors) and (agricultural) establishments using vegetable material (grass, straw, hay and the like) in feeding animals. The ciliate system will require specific adaptation to these substrates; in all these uses, however, the conversion of the plant material into methane (in which a substantial part of the energy is saved) will be much preferred to composting (with a substantial loss of energy), combustion or discharge to surface waters, thereby polluting the environment.

Accordingly, the material concerned in the first instance is vegetable debris, especially debris with a high content of cell wall polymers, which the system according to this invention is capable of degrading very well. One example is pulp from papermills, with a high content of lignocellulose. Agricultural debris, too, for example, from cattle or poultry raising farms, is a suitable substrate. The same applies to (wayside) grass, domestic refuse, and generally all waste material from users of products of photosynthesis.

In addition, the ciliate system can be used for the conversion of higher or lower plants especially cultured for the supply of energy (via "energy-farming" or "ocean-farming") into methane, thereby to convert solar energy (+carbonate and water) into methane (and oxygen).

If the starting material comprises a non-degradable part which remains as a solid residue, this residue can be utilized further. The non-degradable part of vegetable material, for example, may be rich in lignin, which is an interesting raw material for other purposes (raw material for products built up from phenyl groups; an interesting fuel on account of its high heat of combustion).

The recovery of vitamin $B_{12}$ from the biomass containing methane bacteria is also among the possibilities.

There will now follow a more detailed description of the preferred embodiment comprising two spatially separated phases. By using two separate phases, it is possible to create optimum conditions for the different microbial populations.

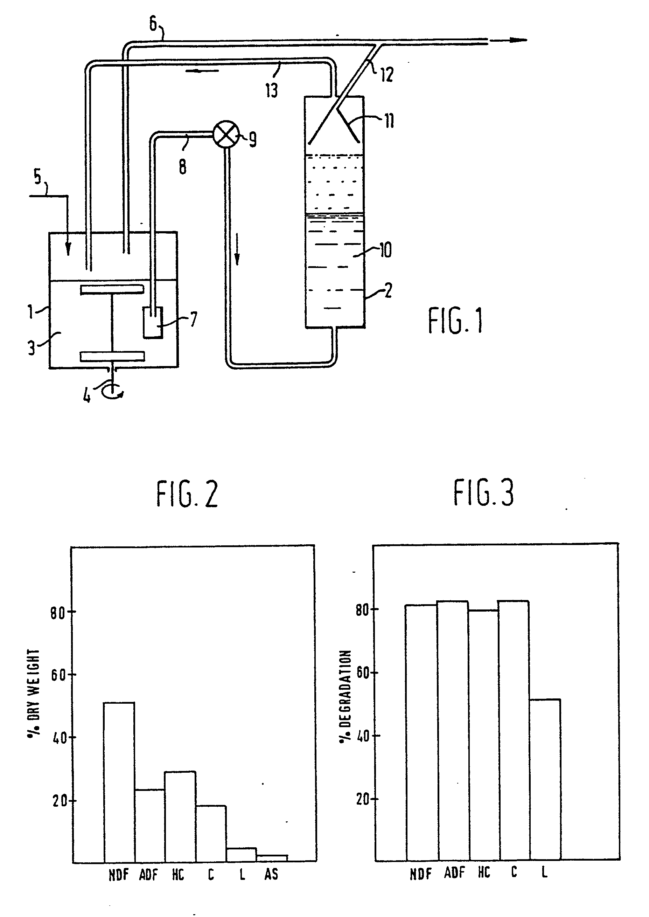

Figure 1 shows the two-phase system diagrammatically.

In the first phase, solid vegetable materials are converted by means of ruminal micro-organisms (ciliates, bacteria, flagellates) into volatile fatty acids (acetate, propionate and butyrate), methane and $CO_2$. This first phase takes place in reactor 1.

In the second phase, the liquid from the first phase, which contains the volatile fatty acids formed, is subjected to such a treatment that the fatty acids are at least partially decomposed and further quantities of methane and $CO_2$ are produced. This second phase takes place in reactor 2.

Reactor 1 contains a reaction mass 3 consisting of liquid and solid constituents, which is stirred by means of a stirrer 4. The solid vegetable material to be digested is supplied through conduit 5. The gases formed during the decomposition (methane and $CO_2$) are removed from reactor 1 through conduit 6. By means of a liquid/solid separator 7, for example, a filter, liquid is removed from reactor 1. This liquid is passed through line 8 to reactor 2 by means of a pump 9. The treatment of the reaction mass 10 in reactor 2 leads to a further production of methane and $CO_2$, which by means of a gas collector 11 and conduit 12 are removed from reactor 2. The liquid treated is recycled to reactor 1 through conduit 13.

a. The first phase

In the first phase, preferably in a continuous process, solid vegetable materials are converted by means of, preferably, ruminal micro-organisms into volatile fatty acids (acetate, propionate and butyrate), methane and $CO_2$.

Highly characteristic and unique as regards use for this phase is the composition in species of the micro-organisms effecting the degradation of vegetable material. As a microbial inocculation, preferably ruminal fluid is used. Typical of the system is specifically the presence of large numbers ($50—150 \times 10^3$/ml) of ruminal ciliates, unicellular animals which have not been described to occur in other places in nature. The composition in species varies with the available nutrients and may comprise the following ciliate species, among others:

1. *Eudiplodinium maggii*
2. *Diplodinium dentatum*
   syn. *Diplodinium denticulatum f. anacanthum*
   syn. *D. denticulatum f. monacanthum*
3. *Epidinium ecaudatum*
   syn. *Epidinium ecaudatum f. hamatum*
4. *Entodinium simplex*
5. *Entodinium longinudeatum*
6. *Entodinium caudatum*
7. *Dasytricha ruminantium*
8. *Isotricha prostoma*

In addition to ciliates flagellates and about 200 different bacterial species are to be found in the first phase. Together the micro-organisms highly effectively convert the vegetable material. Owing to the use of ruminal micro-organisms, the optimum reactor temperature is about 40°C.

As products of degradation are formed: volatile fatty acids, hydrogen and carbon dioxide. Hydrogen and carbon dioxide can be converted into methane by the methane bacterium that is most abundant in the first phase, *Methanobrevibacter ruminantium*, or by other methanogenic bacteria. The above bacterium is found as a free-living organism in the fluid, but characteristic is in addition its high degree of association with various ciliate species, and in particular with *E. maggii* and *D. dentatum*. The first phase hardly contains methane bacteria capable of converting volatile fatty acids into methane. These fatty acids are converted to methane in the second phase. The biogas production of the first phase is about 1—2 1/1 reactor contents per day, and is composed of about 50% methane and about 50% $CO_2$.

A second feature of the first phase is the separation that is made between the liquid and the solid fractions. This separation is needed to effect different residence times of these fractions in the reactor, and this for the following reasons:

1. Owing to the fatty acid production, the pH is lowered. As degradation stops when the pH is too low (pH<5.5), the fatty acids formed must be continuously removed. To keep the pH above its minimum value, a liquid replacement of several (about 2) fermenter volumes per day is necessary;

2. No solid vegetable material must be carried along with the fatty acids removed, since this would be withdrawn from the degradation process;

3. No ciliates must be carried along either, because otherwise the ciliates would rapidly disappear from the first phase, thereby adversely affecting the degradation process. The removal of bacteria during this replacement of liquid is automatically compensated for by the rapid multiplication of these organisms.

On a laboratory scale, the separation between liquid stream and solids stream is brought about by using filtration, for example, by means of a filter having a pore size of 0.03 mm. On a larger scale, other separation systems are more suitable. For this purpose, use can be made of the separation which often occurs naturally between liquid and solid fractions as a result of the formation of flotation and/or settling layers.

When the vegetable material used is of such a nature that such a separation does not materialize, it may be desirable to use centrifugation techniques.

The residence time of the solid fraction depends on the overall degradability, but is in the order of one to several days. In the case of material that is completely degradable, no residue is formed, and the residence time is in fact equal to the degradation period. When the material also contains a non-degradable fraction, this will have to be removed from the fermenter at regular intervals to prevent accumulation.

In summary, the first phase is characterized by the following points.

1. The composition of the micro-organism. Ruminal micro-organisms and specifically ciliates are here used for the first time in the degradation of plant debris.

2. The separation between the solids stream and the liquid stream; the liquid stream is considerably higher than the stream of solids.

3. Optimum pH is 5.5—7.

4. Optimum temperature is 40°C.

5. $H_2$ and $CO_2$ and (possibly formate) are virtually the only substrates for the production of methane.

b. The second phase.

As stated before, the fatty acids formed in the first phase are preferably converted in a second phase into methane and $CO_2$. There are various reasons for doing this:

1. After the removal of fatty acid, the fluid can be passed through the first phase again, so that a closed system is formed (see Figure 1). In the first place this has a cost-saving effect, because buffer capacity is recovered, but in addition it has a beneficial effect on the efficiency of the overall degradation, because in this way the bacteria are not removed from the first phase, but continually recycled to the first phase.

2. Total methane production is increased by a factor of 2—3.

3. Purification on COC basis is higher.

For the second phase, existing systems may be used. Highly suitable are the fluidized-bed system and the Upflow Anaerobic Sludge Blanket (UASB) system. The principle of the first system has been described by Heijnen, $H_2O$ *16*, 266—299 (1983) and Proceedings of the European Symposiom of Anaerobic Waste Water Treatment, TNO Corporate Communication Department, The Hague, 259—274 (1983), and the UASB system has been developed and described in detail by Lettinga et al., Anaerobic Digestion (D. A. Stafford, B. J. Wheatly and D. E. Hughes, eds.) Univ. Coll., Cardiff, Wales, 167—186, (1980).

An important advantage of the process according to the present invention is that the solids residence time is relatively short, in the order of 48 hours, while an extremely high load of 20—40 kg $COC/m^3$. day is possible. This compares very favourably to existing bioreactors, which require residence times of 10—30 days and permit loads on COC basis lower by a factor of 10—20.

In existing two-phase systems, the first phase has a hydrolyzing and acid-forming function only, while the ultimate methane formation takes place in the second phase. In the first phase of the present ciliate system, there is a clear formation of methane in addition to hydrolysis and acid formation.

A further conspicuous feature is that it is not acetate, as in all existing methane fermentation systems, but $H_2O$ and $CO_2$ which are used as precursors for the methane formation in the first phase of this system. Responsible for the methane formation in this phase is especially the ruminal bacterium *Methanobrevibacter ruminantium*. The acetate present is converted by various other methane bacteria in the second phase only. The coupling according to the present invention of the first and the second phases with regard to methane formation is unique (coupling of a hydrogen system with an acetate system).

A comparison with existing solids fermentation systems shows that the system presently proposed is many times (about 10×) more efficient

with regard to residence times, loads and yields of methane. On the ground of the high load in combination with the short residence time, a relatively small plant can be used, while as a result of high methane yields, short payoff periods can be realized.

The invention is illustrated in and by the following examples. The examples concern experiments carried out on a laboratory scale (1.5 l), using grass and paper pulp, respectively, as solid substrates. The experiments described relate to the first phase of the ciliate system only.

Example I

Substrate: dried grass-cereals mixture, mixed in the ratio of 80% grass and 20% cereals (oats, maize, soy flour, wheat flours). Particle size 3 mm. The cell wall composition is shown in the accompanying Figure 2.

The symbols used have the following meanings:

NDF=total cell wall fraction
(cellulose, hemicellulose and lignin)
ADF=cellulose and lignin
HC=hemicellulose
C=cellulose
L=lignin
As=inorganic, insoluble fraction.

Starting up conditions

The experiments described below were all carried out using the same starting-up conditions.

As a microbial inocculation, 0.75 l freshly-tapped rumen fluid (from a sheep having a ruminal fistula) was introduced into the culturing vessel, whereafter the volume was made up with buffer (carbonate/phosphate; pH 7.9) to 1.5 l. Shortly thereafter, the pumps were actuated which provide for a continuous supply of buffer and removal of filtrate, which leaves the culturing vessel via a 0.03 mm filter. Substrate is supplied twice daily, and the removal of homogeneous culture is also effected discontinuously via an overflow level (each half hour for 30 seconds).

The culturing vessels are kept at a temperature of 40°C by means of a hot-water jacket.

After allowing the system to stabilize for 1 to 2 weeks (adaptations of micro-organisms to substrate) decomposition and other parameters (pH, biogas production, volatile fatty acids, ciliate counts) were determined.

One important point with regard to the applicability of the ciliate system is that the ratio of microbial inocculation/reactor volume need not necessarily be 1. If this ratio is 1:100, it is found that after as short a period as 10 days, normal concentrations of the various micro-organisms are present in the culturing vessel. One condition during starting-up is that in this case the culture should be kept free from oxygen.

Experiments

The buffer stream in the culture vessel, expressed in a D value (vat cycles per day) determines the fluid residence time, while the solids residence time can be calculated from the discharge of homogeneous culture (residence time (in hours)=vat volume/overflow volume per hour).

The retention of the ciliates present in the culture vessel is coupled, on the ground of their size (30—150/µm) to the solids stream, whereas bacteria leave the vessel with both the liquid phase and the solid phase.

First experiments showed that, with loading rates of 20 to 25 g grass (dry weight) per l of culture volume per day and a solids residence time of 2 days, good conversions were attained.

Using known assay methods, the degree in which the various fractions of the plant cell wall were degraded was determined. The results of the above experiment, shown in Figure 3, show that approximately 80% of the added cellulose and hemicellulose is decomposed. Data concerning pH, biogas production, ciliates concentration and volatile fatty acid production, associated with Figure 3, were as follows:

loading rate—24 g/l. day
pH—6.5—7.0
$CH_4$ production—1.25 l/l. day
$CH_4/CO_2$ ratio—approximately 1
ciliates count—$60.10^3$/ml
production volatile fatty acids—6—7 g/l. day
D (=dilution or number of vat cycles per day)—1.7 vat volume/day.

The fatty acids present in the culture vessel are mainly acetate, propionic acid and butyric acid, and are formed in the ratio of 3:1:1 (by weight).

When the loading rate is increased to 35 g grass/l. day, with the same D value and residence time, still approximately 75% of the cellulose and hemicellulose is decomposed. With regard to the loading rate, instead of g dry weight, the more current unit of COC may be read, as 1 g grass corresponds to 1 g COC.

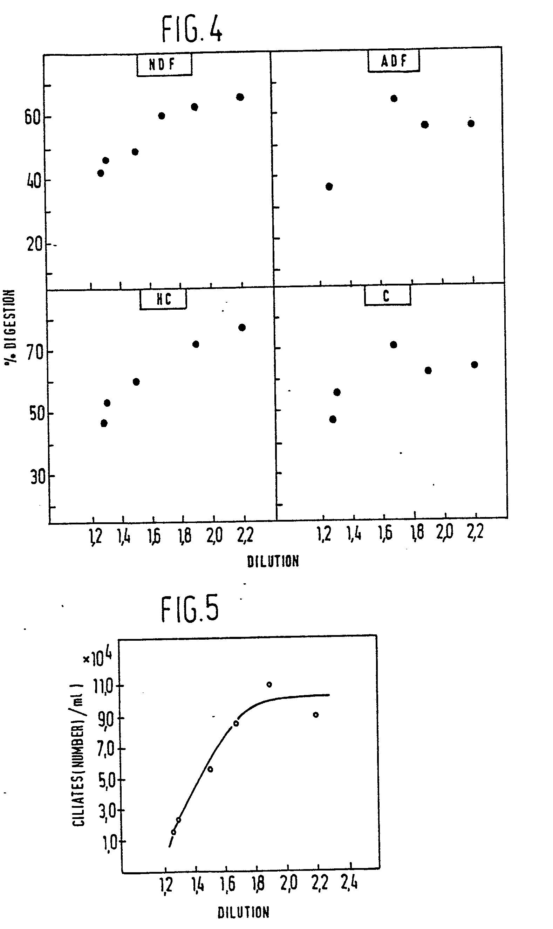

Experiments carried out at different D values show that there is a relationship between the magnitude of the buffer stream and degradation. The relationship between the two is shown in Figure 4. Figure 4 shows that when the Day value is increased from 1.2 to 2.2, degradation of the various cell wall constituents is found to be nearly twice as high.

A virtually comparable relationship is to be found between the ciliates count in the culture vessel and the D value as shown in Figure 5.

These experiments were all carried out using the same loading rate (35 g/l. day) and solids residence time (60 hours). In addition, experiments were run using different solids residence times, with D values (2.0) and loading rates being kept at a constant level. The results of these experiments show that an increase in solids residence time leads to an increase in degradation percentage. When the discharge of solids is stopped altogether, decomposition percentages as high as 90% or higher are even measured. A high D value in combination with long residence times of the solid material will, on the ground of the above data, lead to the best results. In this connection it should be noted that it is not possible to fully stop the removal of solids, or non-degradable or poorly-degradable constituents (lignin, inorganic material) would accumulate in the reactor. Reactor set-up on a technical scale is preferably designed so that a separation can be made in the reactor between fresh and spent material, so that the fraction of solids to be removed is relatively rich in poorly-degradable components.

Example II

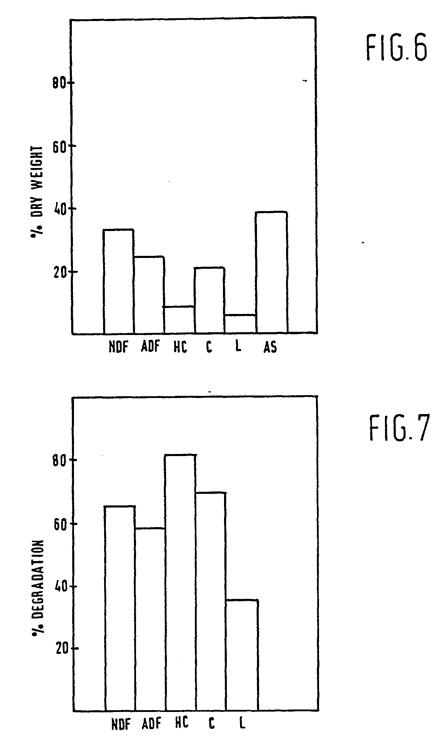

Substrate: Paper pulp from a paper mill. The waste consists as to about 40% of inorganic material (kaolin and chalk). The remaining 60% is principally cellulose (see Figure 6). 1 g of dry weight corresponds to 0.7 g COC.

Starting-up conditions: see Example I.

During the first days after starting the cultures, there is a gradual transition from grass feed to paper pulp, to permit gradual adaptation.

After the experiments with grass had shown under what conditions high ciliates concentrations in combination with good degradation can be realized, we changed to the use of paper pulp for the substrate.

As this refuse consists virtually exclusively of a carbon source, it is necessary to add extra nitrogen and trace elements (essential for microbial growth). In the experiments here described, these components are added to the buffer, while we also added small quantities of grass. On a practical scale, the use of fertilizers or other components as a nitrogen source may be considered.

Experiments with the paper pulp showed that even at very short residence times of the solid material (35 hours) and extremely high loading rates (of up to about 65 g COC/l. day) very good degradation efficiencies can be reached (Figure 7). The data associated with Figure 7 are:

loading rate:

| | | |
|---|---|---|
| 64 g pulp/l. day | = | 47 g COC/l. day |
| 19.5 g grass/l. day | = | 19.5 g COC/l. day |
| | | 66.5 g COC/l. day |

D—2.0 vat volumes/day
pH—6.4—6.7
$CH_4$ production—more than 1.5 l/l. day
$CH_4/CO_2$ ratio—about 1
ciliates count—(100—150) · $10^3$/ml
production volatile fatty acids—more than 7.5 g/l. day, and in addition other acid and neutral products

Degradation percentages for cellulose and hemicellulose in such an experiment were 70% and 80%, respectively.

When the solids residence time is prolonged, however, it is found, unlike earlier measurements in grass cultures, that the degradation efficiency decreases markedly. This is probably a result of the accumulation of inorganic fraction in the culture vessel.

Measurements showed that when the residence time is lengthened, inorganic material accumulates fast up to as much as 175 g/l culture. Comparable experiments were also conducted using filter paper as the substrate. Filter paper, like grass, hardly contains inorganic material. Prolonging the solids residence time with this substrate too, results in better degradation. When the removal of solids is stopped altogether even more than 90% of the filter paper (mainly cellulose) is decomposed.

From the results it can be concluded that paper pulp can be very efficiently converted, provided care is taken that inorganic material is selectively removed, so that higher degradation percentages can be realized with longer residence times.

When filtering paper or paper pulp is added to the culture, there is a considerable shift in ciliates population. After about 7 days, it is especially the larger species (*Eudiplodinium maggii* and *Diplodinium dentatum*) that have become predominant. This is in contrast to grass-fed cultures, where *Entodinium* and *Epidinium* species predominate.

## Claims

1. A process for producing methane from solid vegetable material, characterized by contacting the solid vegetable material in a reactor with a liquid system containing at least one species of ciliates and at least one species of methane bacteria, and removing a methane and $CO_2$ containing gas from the reactor.

2. A process according to claim 1, characterized by using at least one of the following species of ciliates:
    1. *Eudiplodinium maggii*
    2. *Diplodinium dentatum*
       syn. *Diplodinium denticulatum f. anacanthum*
       syn. *D. denticulatum f. monacanthum*
    3. *Epidinium ecaudatum*
       syn. *Epidinium ecaudatum f. hamatum*
    4. *Entodinium simplex*
    5. *Entodinium longinudeatum*
    6. *Entodinium caudatum*
    7. *Dasytricha ruminantium*
    8. *Isotricha prostoma*

3. A process according to claim 1 or 2, characterized by using at least the methane bacterium species *Methanobrevibacter ruminantium*.

4. A process according to any of claims 1—3, characterized by using as the liquid system fluid (inoculating material) from the rumen of ruminants or the cecum of herbivores.

5. A process according to any of claims 1—4, characterized in that the pH is maintained at a value of 5.5—7.

6. A process according to any of claims 1—5, characterized in that the temperature is maintained at a value of 35—45°C.

7. A process according to claim 6, characterized by keeping the temperature at about 40°C.

8. A process according to any of claims 1—7, characterized by continuously or discontinuously removing liquid from the reactor and replacing it with liquid not containing carboxylic acids or a lower proportion thereof.

9. A process according to claim 8, characterized by removing and replacing at least 2 reactor volumes of fluid per day.

10. A process according to claim 8 or 9, characterized by subjecting the fluid removed from the reactor in a separate reactor to a treatment in which the carboxylic acids present in the fluid are decomposed to form methane and $CO_2$, which are removed from said second reactor, and recycling or removing the fluid thus treated to said first reactor.

11. A process according to any of claims 1—10, characterized in that any remaining solid residue, which is rich in non-degradable or poorly-degradable components, is removed from said first reactor after a solids residence time of 1—3 days, and either dumped, or burnt, or utilized for winning valuable substances or for fertilization.

## Patentansprüche

1. Verfahren zur Herstellung von Methan aus einem festen Pflanzenmaterial, dadurch gekennzeichnet, daß das feste Pflanzenmaterial in einem Reaktor mit einem Flüssigkeitssystem, das mindestens eine Species der Ciliaten und mindestens eines Species der Methanbakterien enthält, in Kontakt gebracht wird und ein Methan und $CO_2$ enthaltendes Gas aus dem Reaktor entfernt wird.

2. Verfahren nach Anspruch 1, dadurch gekennzeichnet, daß mindestens eine der folgenden Species der Ciliaten verwendet wird:
1. Eudiplodinium maggii
2. Diplodinium dentatum
   syn. Diplodinium denticulatum f. anacanthum
   syn. D. Denticulatum f. monocanthum
3. Epidinium ecaudatum
   syn. Epidinium ecaudatum f. hamatum
4. Entodinium simplex
5. Entodinium longinudeatum
6. Entodinium caudatum
7. Dasytricha ruminantium
8. Isotricha prostoma.

3. Verfahren nach Anspruch 1 oder 2, dadurch gekennzeichnet, daß mindestens die Methanbakterien - Species Methanobrevibacter ruminantium verwendet wird.

4. Verfahren nach einem der Ansprüche 1 bis 3, dadurch gekennzeichnet, daß als Flüssigkeitssystem das Fluid (Inoculationsmaterial) aus dem Pansen von Wiederkäuern oder aus dem Cöcum von Pflanzenfressern verwendet wird.

5. Verfahren nach einem der Ansprüche 1 bis 4, dadurch gekennzeichnet, daß der pH-Wert bei 5,5 bis 7 gehalten wird.

6. Verfahren nach einem der Ansprüche 1 bis 5, dadurch gekennzeichnet, daß die Temperatur bei einem Wert von 35 bis 45°C gehalten wird.

7. Verfahren nach Anspruch 6, dadurch gekennzeichnet, daß die Temperatur bei etwa 40°C gehalten wird.

8. Verfahren nach einem der Ansprüche 1 bis 7, dadurch gekennzeichnet, daß kontinuierlich oder diskontinuierlich Flüssigkeit aus dem Reaktor entfernt und durch eine Flüssigkeit, die keine Carbonsäuren, oder einen geringeren Mengenanteil davon enthält, ersetzt wird.

9. Verfahren nach Anspruch 8, dadruch gekennzeichnet, daß mindestens zwei Reaktorvolumina Fluid pro Tag entfernt und ersetzt werden.

10. Verfahren nach Anspruch 8 oder 9, dadurch gekennzeichnet, daß das aus dem Reaktor entfernte Fluid in einem getrennten Reaktor einer Behandlung unterzogen wird, bei dem die in dem Fluid vorhandenen Carbonsäuren zersetzt werden unter Bildung von Methan und $CO_2$, die aus dem zweiten Reaktor entfernt werden und daß das so behandelte Fluid in den ersten Reaktor recyclisiert oder entfernt wird.

11. Verfahren nach einem der Ansprüche 1 bis 10, dadurch gekennzeichnet, daß ein evtl. zurückbleibender fester Rückstand, der reich an nichtabbaubaren oder schlecht-abbaubaren Komponenten ist, nach einer Feststoffverweilzeit von 1 bis 3 Tagen aus dem ersten Reaktor entfernt wird und entweder auf der Deponie abgelagert oder verbrannt oder zur Gewinnung wertvoller Substanzen oder zum Düngen verwendet wird.

## Revendications

1. Procédé pour la production de méthane à partir de matière végétale solide, caractérisé par le contact de la matière végétale solide dans un réacteur avec un système liquide contenant au moins une espèce d'infusoires ciliés et au moins une espèce de bactéries de méthane, et l'extraction d'un gaz contenant du méthane et CO2 du réacteur.

2. Procédé selon la revendication 1, caractérisé par l'utilisation d'au moins l'une des espèces suivantes d'infusoires ciliés:
1. Eudiplodinium maggii
2. Diplodinium dentatum
   syn. Diplodinium denticulatum f. anacanthum
   syn. D. denticulatum f. monacanthum
3. Epidinium ecaudatum
   syn. Epidinium ecaudatum f. hamatum
4. Entodinium simplex
5. Entodinium longinudeatum
6. Entodinium caudatum
7. Dasytricha ruminantium
8. Isotricha prostoma.

3. Procédé selon l'une quelconque des revendications 1 ou 2, caractérisé par l'utilisation d'au moins l'espèce de bactérie de méthane Methanobrevibacter ruminantium.

4. Procédé selon l'une quelconque des revendications 1 à 3, caractérisé par l'utilisation comme système liquide (matière d'inoculation) de fluide provenant de la panse des ruminants ou du caecum des herbivores.

5. Procédé selon l'une quelconque des revendications 1 à 4, caractérisé en ce que le pH est maintenu à une valeur de 5,5 à 7.

6. Procédé selon l'une quelconque des revendications 1 à 5, caractérisé en ce que la température est maintenue à une valeur de 35 à 45°C.

7. Procédé selon la revendication 6, caractérisé par le maintien de la température à environ 40°C.

8. Procédé selon l'une quelconque des revendications 1 à 7, caractérisé par l'extraction continuellement ou non de liquide du réacteur et son remplacement par du liquide ne contenant pas d'acides carboxyiliques ou une plus faible proportion de ceux-ci.

9. Procédé selon la revendication 8, caractérisé par l'extraction et le remplacement d'au moins 2 volumes de réacteur de fluide par jour.

10. Procédé selon l'une quelconque des revendications 8 ou 9, caractérisé par la soumission du fluide extrait du réacteur dans un réacteur séparé à un traitement dans lequel les acides carboxyliques présents dans le fluide sont décomposés pour former du méthane et du $CO_2$, qui sont extraits de ce second réacteur et le recyclage ou l'extraction du fluide ainsi traité au premier réacteur.

11. Procédé selon l'une quelconque des revendications 1 à 10, caractérisé en ce que tout résidu solide restant qui est riche en éléments non dégradables ou médiocrement dégradables, est extrait de ce premier réacteur après une durée de séjour des solides de 1 à 3 jours, et soit vidé, soit brûlé, soit utilisé pour en tirer des substances appréciables ou pour la fertilisation.

FIG.1

FIG.2

FIG.3

# FIG. 4

# FIG. 5

FIG.6

FIG.7